# FASCICULE DE BREVET EUROPEEN

(11) **EP 0 374 093 B1**
(45) Date de publication et mention de la délivrance du brevet: **26.07.1995**
(21) Numéro de dépôt: 89810912.9
(22) Date de dépôt: 29.11.1989
(51) Int. Cl.: A61B 17/60

(54) **Bride porte-fiches**
Nagelhaltestück
Pin holder body

(30) Priorité: 15.12.1988 CH 4631/88
(43) Date de publication de la demande: 20.06.1990
(73) Titulaire: JAQUET ORTHOPEDIE S.A., CH-1228 Plan-les-Ouates (CH)
(72) Inventeur: Wagenknecht, Marcel, CH-1219 Le Lignon (CH)
(74) Mandataire: Dietlin, Henri

(56) Documents cités:
- EP-A- 0 314 021
- FR-A- 2 520 607
- FR-A- 2 579 688
- GB-A- 2 077 847
- GB-A- 2 110 094
- US-A- 2 393 694
- US-A- 4 848 368

## Description

La présente invention est du domaine de la traumatologie et de l'orthopédie et a plus précisément pour objet une bride porte-fiches destinée à des appareils externes de fixation osseuse au moyen de fiches introduites dans l'os.

Par traumatologie, on entend la chirurgie des accidents et par orthopédie la correction de malformations, congénitales ou subséquentes à une consolidation osseuse selon un alignement incorrect.

Depuis de nombreuses années on a développé plusieurs dispositifs autorisant le transfert ou le déplacement d'un segment d'os par rapport au reste de celui-ci. Selon les cas, le déplacement nécessité peut être angulaire, longitudinal ou de rotation, ou toute combinaison de ceux-ci.

Les dispositifs utilisés sont principalement de deux types : les dispositifs internes, qui sont introduits dans le corps du patient, et les fixateurs externes, reliés aux fragments d'os à déplacer ou maintenir au moyen de fiches insérées dans l'os.

Ces dispositifs sont toujours mis en place sous anesthésie. Afin que la durée de celle-ci soit minimale, on cherche à développer des éléments offrant tous les positionnements angulaires possibles, mais dont le réglage soit aisé pour un chirurgien, qui n'est pas forcément spécialisé en mécanique.

L'invention se rapporte à des fixateurs externes, reliés à des groupes de fiches insérées dans chacun des fragments d'os à positionner l'un par rapport à l'autre, de part et d'autre de la fracture.

Selon une première technique actuellement utilisée, on dispose des éléments d'arceaux autour du membre, ces arceaux étant solidaires d'une part des fiches ou fils insérés dans l'os et d'autre part de barres d'écartement entre les arceaux. On emploie également des cadres disposés de part et d'autre du membre et reliés par des fiches traversant les fragments d'os.

Selon une autre technique, les fiches sont reliées à une barre de fixation unique, disposée sensiblement parallèlement à l'os fracturé. Chaque groupe de fiches insérées dans un fragment d'os est serré dans un support orientable par rapport à la barre de fixation, et solidaire d'une bride de fixation sur celle-ci.

Afin de permettre l'orientation de chaque groupe de fiches, on connaît différents systèmes à rotules ou joints universels, qui ont pour inconvénient que leur position ne peut pas être modifiée de manière continue.

On a déjà proposé d'utiliser des organes comportant des faces annulaires crantées susceptibles de s'engager dans des éléments correspondants, par rapport auxquels ils sont fixés par des éléments de liaison traversant un alésage central, tel le système décrit dans le brevet français FR-A-2 557 933. L'articulation obtenue avec ce système est néanmoins limitée aux positions angulaires prédéterminées des crans et nécessite de plus un double réglage, dans deux plans, pour obtenir l'orientation désirée, la fixation des fiches étant de plus obtenue par un système de serrage complémentaire.

On a aussi proposé une bride porte-fiches solidaire d'un organe arqué de guidage comme décrit dans le brevet anglais GB-A-2 110 094. L'organe arqué de guidage est doté d'une denture hélicoïdale destinée à coopérer avec une vis sans fin; il est disposé dans un plan parallèle à l'axe de la barre et son rayon correspond à sa distance au centre de l'os. Le mouvement obtenu est limité à un angle de 20 à 25° de part et d'autre d'une position centrale, en raison des formes des composants.

On connait d'autre part aussi un dispositif composé de deux éléments, l'un solidaire des fiches et l'autre de la barre de fixation, l'un des éléments présentant une partie arquée de guidage de l'autre, tel que décrit dans le brevet US-A-4 628 922. Ce dispositif nécessite le serrage indépendant des fiches et le blocage du positionnement des éléments par des organes de serrage indépendants.

La demande de brevet européen EP-A-0 314 021, état de la technique en vertu de l'article 54(3) et (4) CBE, décrit un fixateur présentant plusieurs brides de fixation permettant chacune l'orientation d'une fiche unique, positionnée par rapport à la barre à l'aide de vis indépendantes pour chaque degré de liberté de chaque fiche.

La présente invention propose une bride de fixation d'un groupe de fiches et de positionnement angulaire de celles-ci par rapport à une barre de fixation ou d'écartement, comportant :
- un étau de serrage des fiches,
- une mâchoire de positionnement le long de la barre,
- un organe de déflection disposé entre l'étau et la mâchoire et comportant sur une face des moyens de rotation de l'étau par rapport à la mâchoire, et sur la face opposée des moyens arqués de guidage du pivotement de l'étau par rapport à la mâchoire selon un axe de pivotement orthogonal à l'axe de rotation et
- des moyens de blocage dans la position désirée.

Elle est caractérisée en ce que lesdits moyens de rotation coopèrent avec des moyens complémentaires de l'étau ou de la mâchoire pour assurer la libre rotation de 0 à 360° de l'étau par rapport à la mâchoire selon un axe de rotation croisant à 90° l'axe de la barre, lesdits moyens arqués de guidage coopèrant avec des moyens arqués complémentaires de la mâchoire ou de l'étau respectivement, et en ce que lesdits moyens de blocage comportent un assemblage de serrage apte à assurer simultanément le blocage des fiches d'une part et le blocage dudit pivotement ou de ladite rotation d'autre part dans la position finale désirée.

Dans une variante préférentielle, l'étau est constitué par un mors d'étau supérieur et un mors d'étau inférieur présentant un passage central pour ledit assemblage de serrage.

La présente invention propose en outre des moyens de positionnement de la bride le long de la barre de fixation avec, dans une forme d'exécution, un limiteur de serrage et de desserrage. Dans une variante, la bride peut coulisser le long de la barre d'écartement.

L'étau permet de maintenir les fiches dans un plan tout en autorisant leur positionnement angulaire par rapport à une barre de fixation, le long de laquelle elle peut coulisser, cette barre de fixation étant disposée sensiblement parallèlement à l'os.

Pour que la gêne suscitée chez le patient par le fixateur externe soit minimum, on cherche autant que possible à diminuer l'encombrement et le poids de ses composants.

Dans l'exposé, on parle d'os fracturé, mais il va sans dire qu'il peut s'agir aussi d'un os volontairement sectionné, afin de l'allonger par exemple en orthopédie.

Le dessin annexé montre, à titre d'exemple non limitatif, une forme d'exécution de l'objet de la présente invention.

La figure 1 est une vue générale schématique en perspective de brides portant des fiches, montée sur une barre de fixation externe comprenant en outre un dispositif de déplacement latéral de l'une des brides.

La figure 2 représente une bride dans un plan parallèle à l'axe de la barre, les fiches étant orientées perpendiculairement. Les éléments sont vus en coupe dans la moitié droite de la figure 2 et vus de côté dans la moitié gauche.

La figure 3 est une vue de la bride, dans un plan perpendiculaire à l'axe de la barre, vue en coupe dans la moitié gauche de la figure et vue de côté dans la moitié droite.

La figure 4 est une vue en coupe de la bride de la figure 3, à plus grande échelle.

La figure 5 est une vue d'une variante de bride vue dans un plan parallèle à l'axe de la barre, avec coupe partielle, montrant un renvoi angulaire permettant d'orienter les fiches dans d'autres directions.

La figure 6, ne faisant pas partie de l'invention, représente une variante de bride selon l'invention, dans un plan parallèle à la barre, vue en coupe dans la partie gauche, et de côté dans la partie droite.

La figure 7, ne faisant pas partie de l'invention, est une vue transversale à la barre de la bride de la figure 6, en coupe dans la partie droite et vue de côté dans la partie gauche.

La figure 8, ne faisant pas partie de l'invention, est une coupe selon VIII-VIII à la figure 6.

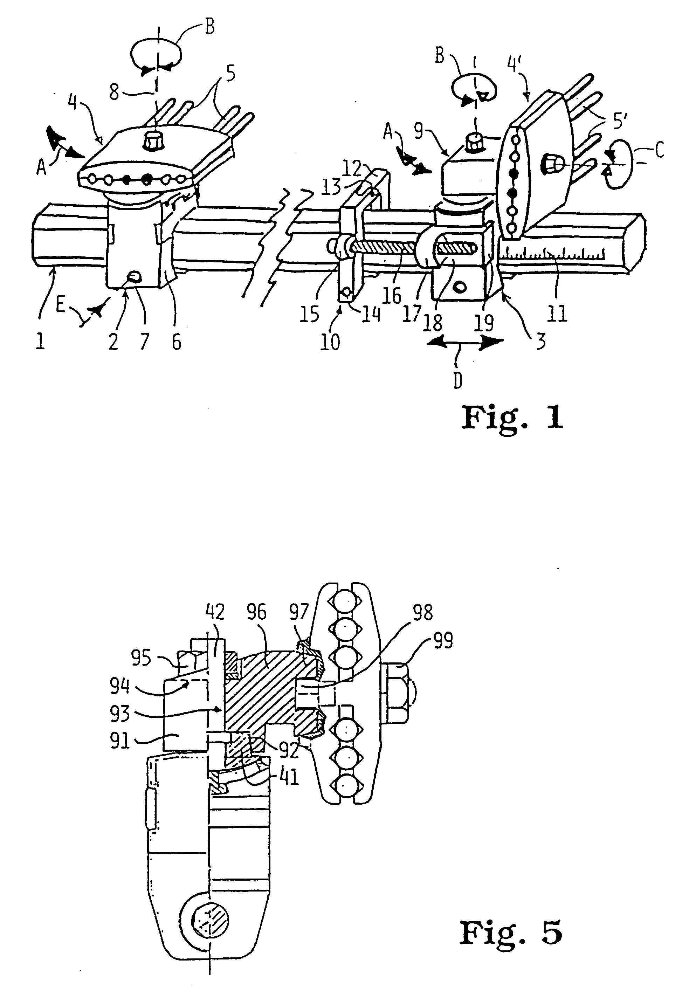

Dans la vue générale de la figure 1 on a représenté une barre de fixation externe 1, de section polygonale, sur laquelle sont placées deux brides 2 et 3 selon l'invention, solidaires indirectement d'un étau 4 de serrage de fiches 5.

Au dessin, on a représenté une barre de fixation externe 1 continue, mais il va de soi qu'en variante la barre peut être constituée par une barre à éléments télescopiques.

Chaque bride comporte un organe arqué de déflection disposé entre l'étau 4 et la mâchoire 6 qui entoure la barre 1 sur laquelle elle est fixée par un dispositif de positionnement 7. Cet organe arqué de déflection n'est pas représenté dans la vue schématique de la figure 1 mais sera détaillé plus loin. Il autorise le pivotement angulaire de l'étau 4 selon la flèche A d'une part, et l'orientation continue de 0 à 360° de l'ensemble des fiches par rapport à l'axe 8 de serrage, selon la flèche B d'autre part.

La bride 3 de la figure 1 comporte en outre un organe de renvoi angulaire 9, permettant de basculer de 90° l'étau 4′ et par conséquent les fiches 5′. Un tel renvoi angulaire est utilisé par exemple lorsque les fiches 5′ sont destinées à être insérées dans l'épiphyse d'un os (par exemple, plateau tibial ou fémoral)dont le corps diaphysaire recevrait les fiches 5. En plus du pivotement circulaire selon la flèche A et de l'orientation angulaire selon la flèche B, la bride 3 permet aussi une orientation angulaire selon la flèche C. Quant à la flèche D, elle représente le déplacement de la bride le long de la barre.

Dans la vue schématique de la figure 1 on a également représenté un organe 10 de déplacement de la bride 3 le long de la barre 1 qui peut comporter des repères linéaires 11, généralement gradué en millimètres.

L'organe de déplacement 10 est constitué d'une bride de fixation 12, en deux parties articulées sur un axe 13, qui est serrée sur la barre 1 par une vis 14. Il comporte en outre un support 15, solidaire en rotation de la bride 12 et présentant une ouverture pour la fixation de la tête d'une vis-mère 16 le long de laquelle se déplace un bossage taraudé 17 venu de fabrication avec un étrier 18 muni de deux ailes 19 destinées à enserrer la partie latérale de la bride 3.

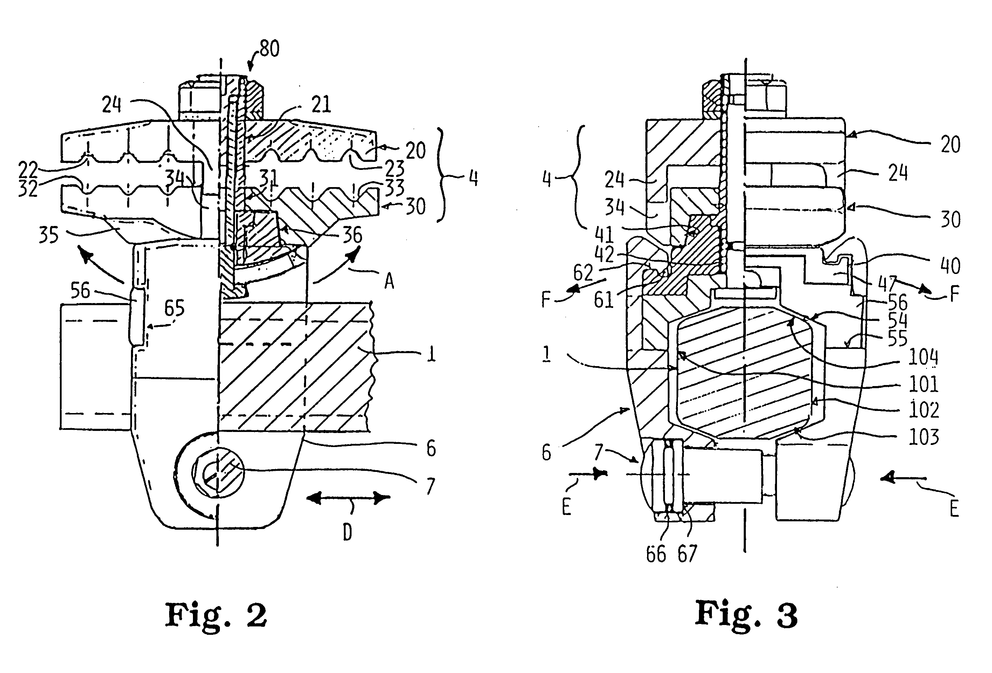

En se référant aux figures 2 et 3, on retrouve la barre de fixation externe 1, l'étau 4, la mâchoire 6, le dispositif de positionnement 7 permettant le blocage de la bride sur la barre 1 ainsi qu'un assemblage 80 de serrage des fiches selon l'axe 8. L'organe arqué de déflection déjà mentionné est représenté sous référence générale 40.

De manière plus détaillée, l'étau 4 est constitué par un mors supérieur 20 et un mors inférieur 30, tous deux de forme générale rectangulaire et présentant un passage central 21, 31 pour l'assemblage de serrage 80. Les surfaces des mors situées en vis-à-vis comportent des rainures parallèles 22, 32, destinées au passage des fiches insérées dans l'os. Il est à noter que les rainures 22 ou 32 présentent dans leur partie centrale un évidement 23 ou 33 destiné à assurer un meilleur ancrage des fiches.

Le mors d'étau supérieur 20 comporte de plus deux tétons latéraux 24 faisant saillie vers le bas et destinés à pénétrer dans des dégagements correspondants 34 du mors inférieur, de manière à empêcher la rotation des mors 20 et 30 l'un par rapport à l'autre.

Le mors inférieur 30 de l'étau comporte, dans sa partie inférieure, un bossage central 35 circulaire muni d'un dégagement central tronconique 36, dont l'angle est de l'ordre de 7°.

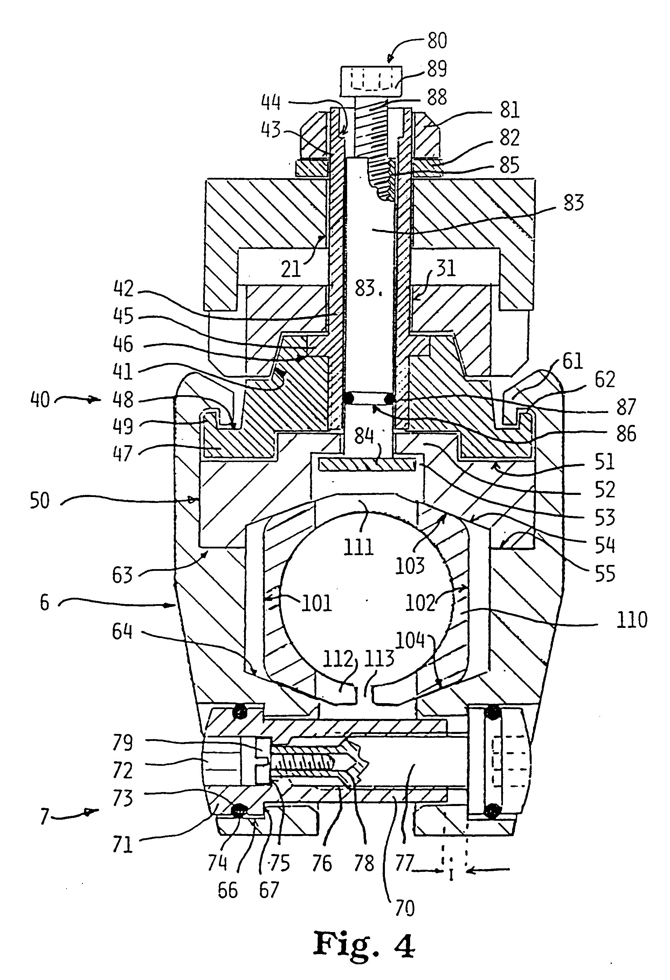

Le dégagement tronconique 36 est destiné à recevoir un épaulement tronconique correspondant 41 de l'organe arqué de déflection 40, dont la partie centrale reçoit un arbre creux 42 présentant un filetage extérieur 43 et un épaulement interne 44 dans sa partie extrême (voir figure 4). L'arbre creux 42 est généralement en acier inoxydable et est scellé dans l'organe arqué de déflection 40 par tout moyen connu de l'homme de métier (vissage ou collage par exemple). Pour assurer le positionnement correct de l'arbre creux 42, on peut prévoir une collerette 45 d'appui dans une ouverture cylindrique correspondante 46 prévue dans la partie supérieure de l'organe de déflection 40. L'arbre creux 42 est destiné à traverser le passage central 31 du mors inférieur 30 et le passage central 21 du mors supérieur 20 de l'étau 4.

L'organe arqué de déflection 40 a un rayon de courbure dont le centre est situé au-delà du plan des fiches. Il comporte dans sa partie inférieure deux patins arqués 47, symétriques et parallèles, présentant chacun une rainure 48 qui délimite un épaulement arqué 49 dans la partie supérieure de chaque patin 47. Comme visible au dessin, les patins arqués 47, et par conséquent les épaulements arqués 49, sont disposés parallèlement à l'axe de la barre.

Les patins arqués 47 sont destinés à glisser le long de creusures 51 de courbure correspondante pratiquées dans un pont 50 disposé entre la double mâchoire 6 formée de deux parties symétriques. La partie centrale 52 du pont est munie d'une ouverture oblongue 53 parallèle aux patins 47.

Dans sa partie inférieure, le pont 50 comporte deux pans inclinés 54, dont la pente correspond à la forme de la barre 1 et deux rebords droits 55, munis à chaque extrémité d'une aile 56 faisant saillie vers l'extérieur (fig. 2 et 3).

Chacune partie formant la mâchoire 6 comporte symétriquement, dans sa partie intérieure visible à la figure 4 :
- un épaulement arqué 61 et une rainure 62 destinés à coopérer respectivement avec la rainure 48 et l'épaulement 49 de l'organe de déflection 40;
- un rebord droit 63 destiné à s'appuyer sur le rebord correspondant du pont 50;
- un pan incliné 64, dont la pente correspond également à la forme de la barre 1.

Comme visible à la figure 2, chaque mâchoire comporte, au-dessus du rebord droit, des dégagements latéraux 65 destinés au logement des ailes latérales 56 du pont 50. Dans sa partie inférieure, chaque mâchoire comporte une ouverture 66 présentant un rebord intérieur 67 destiné à coopérer avec le dispositif de positionnement 7.

Ce dispositif de positionnement 7 sera décrit en se référant à la figure 4 qui est plus détaillée. Il comporte un arbre creux 70 présentant une tête 71 avec une ouverture 72 vers l'extérieur destinée à coopérer avec un outil de serrage, par exemple une ouverture à 6 pans. Le diamètre de la tête 71 est tel qu'elle pénètre dans l'ouverture 66 de la mâchoire et vienne s'appuyer sur le rebord intérieur 67. On remarquera que la tête 71 comporte en outre une rainure circulaire extérieure 73 destinée à recevoir un anneau de type O-ring 74.

L'arbre creux 70 comporte un épaulement intérieur 75 et se termine par un taraudage central 76.

Le dispositif de positionnement 7 comporte également un arbre central 77 présentant une tête 71 pareille à celle décrite auparavant. L'arbre central 77 comporte un filetage extérieur destiné à coopérer avec le taraudage 76 de l'arbre creux 70 et un taraudage intérieur 78 destiné à recevoir une vis 79 à tête cylindrique.

On remarquera à la figure 4 que lorsque la tête de la vis 79 vient buter contre l'épaulement intérieur 75, il subsiste un intervalle i entre l'extrémité de l'arbre creux 70 et la face interne de la tête 71 de l'arbre central 77.

L'assemblage de serrage 80 est constitué par un écrou 81, monté sur une rondelle 82 et destiné à être vissé sur le filetage 43 de l'arbre creux 42, solidarisé comme on l'a déjà vu de l'organe arqué de déflection 40.

Dans la forme d'exécution représentée à la figure 4, on a disposé à l'intérieur de l'arbre creux 42 un tube central 83. Ce tube central 83 est terminé dans sa partie inférieure par une plaquette 84 et dans sa partie supérieure par un taraudage central 85. La plaquette 84 est fixée en bout du tube 83 par tout moyen connu de l'homme du métier. Elle présente une forme arquée dont la courbure correspond à celle de la partie centrale 52 du pont 50 contre laquelle elle vient s'appuyer tandis que le tube 83 traverse librement l'ouverture oblongue 53 du pont, avant de pénétrer dans l'arbre creux 42.

Le tube 83 présente en outre une rainure 86 pour l'insertion d'un anneau de type O-ring 87. L'assemblage de serrage 80 est complété par une vis 88 dont la tête 89 présente une ouverture à 6 pans et est dimensionnée de manière à s'appuyer contre l'épaulement interne 44 de l'arbre creux 42.

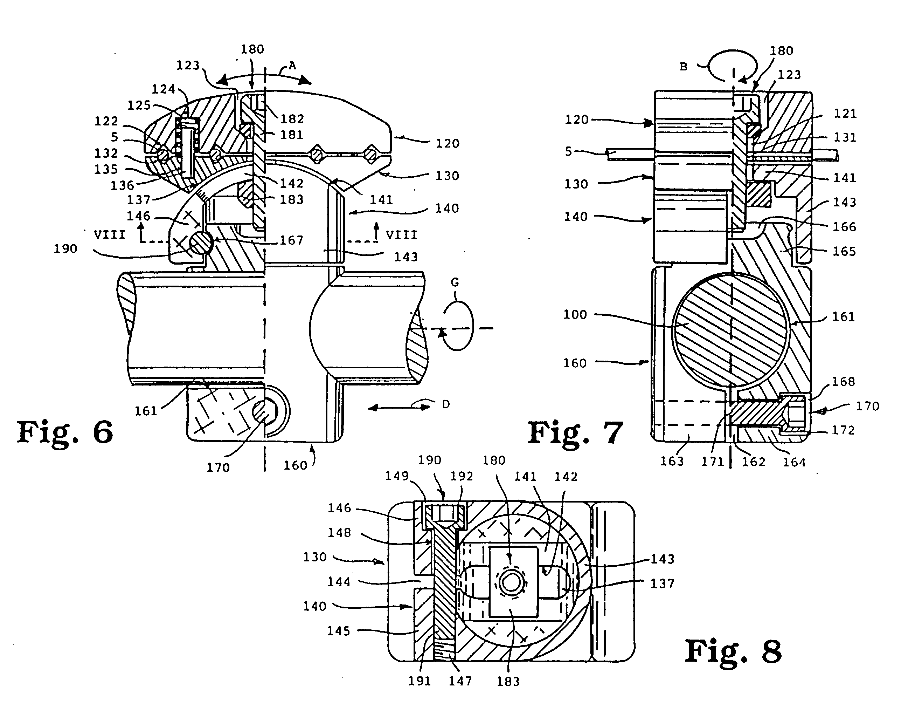

Le renvoi angulaire 9 déjà schématisé dans la figure 1 est représenté en détail à la figure 5. C'est une pièce allongée présentant à sa base une collerette 91 définissant une ouverture tronconique 92 de forme correspondant à l'épaulement conique 41 de l'organe arqué de déflection 40. Au centre de la collerette 91 une ouverture de part en part 93 permet le passage de l'arbre creux 42 et débouche sur un plat 94 destiné à coopérer avec un écrou de serrage 95. Une protubérance 96 s'étend perpendiculairement à l'ouverture 93 et se termine par un épaulement tronconique 97, destiné à coopérer avec le dégagement tronconique 36 pratiqué dans le mors 30 de l'étau de serrage des fiches. Au centre de l 'épaulement 97 est fixée une tige filetée 98, par tout moyen connu. La tige filetée 98 est destinée à traverser l'étau 4′ de fixation des fiches 5′ dans un plan transversal par rapport à la barre d'écartement, et vient à cet effet en prise avec un écrou 99.

Comme on l'a déjà mentionné, la barre de fixation externe 1 est de section polygonale. Dans la forme d'exécution décrite ici, la barre représentée à la figure 3 comporte deux faces parallèles, 101 et 102, disposées entre la paire de mâchoires 6, chacune de ces faces étant suivie par une face 103 et 104, inclinée de 20° de manière à suivre la forme du pan incliné 54 du pont et du pan incliné 64 de chaque mâchoire correspondants.

Dans la variante de la figure 4, on remarquera que la bride selon l'invention peut également être fixée sur une barre cylindrique. A cet effet, on rajoute une pièce intermédiaire 110 entre les mâchoires, dont les parois extérieures correspondent aux faces parallèles 101 et 102 ainsi qu'aux faces inclinées 103 et 104 déjà décrites. Le raccord 110 comporte deux ouvertures 111 et 112, de forme générale rectangulaire et dimensionnées pour autoriser le passage de la plaquette 84 fixée en bout du tube 83. Une fente 113 est ménagée sur toute la longueur du raccord 110, de manière à donner à celui-ci une certaine élasticité. De plus, de chaque côté de la paire de mâchoires, le raccord 110 comporte une collerette vers l'extérieur, non représentée au dessin, destinée à le maintenir dans la bride.

En se référant aux figures 6 à 8 qui présentent une autre forme d'exécution, on trouve une barre de fixation externe 100, de section circulaire, un étau constitué par un mors supérieur 120 et un mors inférieur 130, une mâchoire 160 de positionnement de la bride le long de la barre, un dispositif de blocage 170 permettant le positionnement de la bride le long de la barre 100 ainsi qu'un assemblage 180 de serrage des fiches 5. L'organe arqué de déflection est représenté sous référence générale 140; il comporte un assemblage 190 de fixation angulaire par rapport à la mâchoire 160.

De manière plus détaillée, les mors supérieur 120 et inférieur 130 de l'étau sont tous deux de forme générale rectangulaire et présentent un passage central 121, 131 pour l'assemblage de serrage 180. Les surfaces des mors situées en vis-à-vis comportent des rainures parallèles 122, 132, destinées au logement des fiches 5 insérées dans l'os.

Le mors d'étau supérieur 120 comporte, dans sa partie supérieure, une creusure 123 de logement de la tête de vis de serrage 180 et, dans sa partie inférieure deux dégagements symétriques 124 destinés à empêcher la rotation des mors 120 et 130 l'un par rapport à l'autre, comme on le verra par la suite.

Le mors d'étau inférieur 130 comporte, dans sa partie supérieure, deux dégagements 135 destinés à recevoir deux tétons 136 faisant saillie dans les dégagements 124 du mors supérieur qui reçoivent en outre deux ressorts 125 tendant à écarter les mors 120 et 130 l'un de l'autre pour faciliter l'introduction des fiches 5. Le mors inférieur 130 comporte en outre, dans sa partie inférieure, une creusure cylindrique 137 pour permettre le pivotement de l'étau et par conséquent des fiches par rapport à l'organe de déflection 140.

Cet organe de déflection 140 est constitué par une calotte dont le fond 141 présente une courbure correspondant à la creusure cylindrique 137 du mors inférieur. Le fond 141 présente en outre une ouverture oblongue 142 laissant libre passage à l'assemblage 180 de serrage. La paroi 143 de la calotte est de forme générale cylindrique et comporte une fente 144 en prolongement de l'une des extrémités de l'ouverture 142, cette fente étant ménagée entre deux ailes 145 et 146, en saillie vers l'extérieur.

Dans la coupe de la figure 8, on notera que la mâchoire 160 n'est pas dessinée afin de permettre la représentation de l'organe de déflection 140. L'aile 145 comporte une ouverture taraudée 147 et l'aile 146 comporte un passage 148 débouchant extérieurement dans un dégagement extérieur 149 pour l'introduction de l'assemblage de fixation 190 disposé transversalement à l'ouverture oblongue 142 et à la fente 144.

Dans cette forme d'exécution la mâchoire 160 est réalisée en une seule pièce, de forme générale cylindrique, présentant un passage transversal 161 pour la barre de fixation 100, prolongé par une fente 162 délimitant deux ailes 163 et 164 et permettant le serrage de la mâchoire 160 sur la barre 100 au moyen du dispositif de serrage 170 autorisant le positionnement de la bride le long de la barre de fixation. Comme visible à la figure 7, la mâchoire 160 présente un rebord supérieur circulaire 165 destiné à coopérer avec la paroi 143 et sur lequel l'organe de déflection 140 peut être fixé au moyen de l'assemblage de fixation 190. Vers le haut, le rebord 165 comporte un dégagement 166 pour l'assemblage de serrage 180. Extérieurement le rebord 165 présente une gorge 167 destinée au passage de l'assemblage de fixation 190, pour solidariser l'organe de déflection 140 de la mâchoire 160, et disposée sur une partie de la circonférence pour des raisons qui seront explicitées plus loin.

Le dispositif de serrage 170, visible en coupe à la figure 7, est disposé perpendiculairement à la fente 162 et sert à rapprocher les ailes 163 et 164. Il est constitué par une vis 171 dont la tête 172 est noyée dans une creusure 168 de l'aile 164 et dont la partie filetée traverse l'aile 164 avant de venir en prise dans un taraudage pratiqué dans l'aile 163.

L'assemblage de serrage 180 des figures 6 et 7 est constitué par une vis 181 dont la tête 182 est destinée à appuyer contre la creusure 123 pratiquée dans le mors supérieur 120. La partie filetée de la vis 181 traverse les passages 121 et 131 pratiqués dans les mors supérieur et inférieur, passe dans l'ouverture oblongue 142 et vient en prise dans un plot 183, dont la face supérieure épouse la forme de la courbure du fond 141 de l'organe de déflection.

L'assemblage de fixation 190, visible en coupe à la figure 8, est constitué par une vis 191 dont la tête 192 est destinée à appuyer contre le dégagement 149 de l'aile 146. La partie filetée de la vis 191 traverse librement le passage 148 et vient en prise dans l'ouverture taraudée 147 de l'aile 145.

Il est à noter que les têtes de vis 172, 182 et 192 présentent toutes la même ouverture hexagonale, pour pouvoir être serrées avec un outil unique. De plus certaines de ces têtes de vis pourront comporter un crantage sur leur périphérie, pour faciliter un pré-serrage manuel.

Pour le bien-être du patient, les différentes pièces constituant la bride selon l'invention (telles que l'étau de serrage des fiches, l'organe de déflection, les mâchoires et l'organe de renvoi angulaire) ne présentent pas d'arêtes vives et sont réalisées en aluminium ou autre alliage léger afin de limiter le poids de l'ensemble.

Avant d'être utilisée au cours de l'opération, la bride selon l'invention est assemblée de la manière représentée au dessin, avec ou sans organe de renvoi angulaire selon les besoins.

Au cours de l'opération proprement dite, le médecin insère des groupes de fiches dans chaque fragment d'os, selon les techniques connues. Dans la forme d'exécution représentée aux figures 2 à 5, chaque groupe de fiches 5 est maintenu dans un plan au moyen de l'étau 4 et est positionné par rapport à la barre 1 en profitant des possibilités d'orientation continue de l'étau selon la flèche B, grâce au positionnement relatif du dégagement tronconique 36 du mors 30 de l'étau et de l'épaulement tronconique correspondant 41 de l'organe arqué de déflection 40. On bloque alors l'écrou 81 sur le filetage extérieur 43 de l'arbre creux 42.

Le blocage du pivotement angulaire de l'étau selon la flèche A peut être réalisé de deux manières.

Premièrement, dans les cas où la bride selon l'invention est destinée à être fixée sur la barre de fixation 1, il suffira de serrer le dispositif de positionnement 7 sur la barre pour obtenir à la fois la fixation de la bride et le blocage angulaire par rapport à la flèche A. En effet, en agissant sur l'une des tête de vis 71, on resserre la paire de mâchoires 6 selon les flèches E à la figure 3. Le glissement du pan incliné 64 de chaque mâchoire sur la face inclinée 103 de la barre déplace la partie supérieure de la mâchoire selon la flèche F (figure 3). Par conséquent, l'épaulement arqué 61 de la mâchoire vient s'appuyer contre l'épaulement arqué 49 correspondant de l'organe de déflection 40 qui est ainsi bloqué.

Dans le second cas, où la bride est destinée à pouvoir coulisser le long de la barre d'écartement, les mâchoires 6 ne seront donc pas serrées contre celle-ci. Pour obtenir le blocage du pivotement selon la flèche A de l'organe de déflection 40, on agit sur la vis 88 dont la tête 89 vient s'appuyer contre l'épaulement interne 44 de l'arbre creux 42 et permet de déplacer le tube central 83 vers le haut. Comme on l'a déjà dit, le tube 83 est solidaire d'une plaquette 84 s'appuyant sur la partie centrale 52 du pont et autorisant ainsi le serrage de ce pont 50 par rapport à l'organe arqué de déflection 40, qui se trouve ainsi immobilisé.

L'anneau de type O-ring 87 disposé autour du tube central 83 a pour mission de retenir celui-ci à l'intérieur de l'arbre creux 42 tant que la vis 88 n'est pas mise en place.

On a déjà mentionné précédemment que la tête 79 de la vis 78 du dispositif de positionnement 7, quand elle est bloquée dans l'arbre central 77, vient s'appuyer contre l'épaulement intérieur 75 de l'arbre creux 70, dans la disposition représentée à la figure 4, qui laisse subsister un intervalle i entre l'extrémité de l'arbre creux 70 et la face interne de la tête 71 de l'arbre central 77.

Cette configuration a pour but d'éviter un serrage excessif des mâchoires sur la barre. En effet, lorsque le médecin agira pour serrer l'une des têtes de vis 71 de l'arbre creux 70 ou de l'arbre central 77, il ne pourra opérer qu'un déplacement annulant l'intervalle i. Dès que l'extrémité de l'arbre creux 70 bute contre la tête de l'arbre central 77, tout le dispositif de positionnement 7 tourne librement dans les ouvertures 66 des mâchoires, grâce à la présence des anneaux O-rings 74. Lorsque l'on veut desserrer le dispositif de positionnement 7, il suffit d'agir sur l'une des têtes de vis 71 : le joint O-ring 74 de l'autre ensemble reste bloqué, car le couple de résistance du joint O-ring est supérieur au couple de dévissage. On a ainsi un limiteur de desserrage grâce auquel les mâchoires de la bride restent dans une position telle que la bride glisse le long de la barre. Le déplacement possible de l'intervalle i correspond à environ deux tours.

Dans la forme d'exécution des figures 6 à 8, la mise en place a lieu de manière analogue à celle décrite précédemment. Les fiches 5 sont maintenues dans un plan au moyen de l'étau formé par les mors 120 et 130. Il est à noter que les ressorts 125 ont tendance à appuyer la creusure 137 sur le fond arqué 141, ce qui freine le déplacement de l'étau sur l'organe de déflection.

Avec un assemblage de ce type, le médecin peut régler séparément le pivotement de l'étau par rapport à l'organe de déflection, selon la flèche A, et la rotation de l'ensemble constitué par l'étau et l'organe de déflection par rapport à la mâchoire 160, selon la flèche B. Pour faciliter le travail du praticien, on a cherché à disposer les têtes 172 et 192 des vis 171 et 191 du même côté de la bride. C'est dans ce but que l'on dispose sur une partie de la circonférence du rebord 165 comprise entre environ 90 à 180° la gorge 167 autorisant le passage de l'assemblage de fixation 190. La rotation selon la flèche B de l'étau par rapport à l'organe de déflection est alors limitée entre 0 et environ 90 à 180°.

On notera encore que toute la bride peut être déplacée le long de la barre 100 selon la flèche D et pivoter par rapport à cette barre selon la flèche G tant que le dispositif de serrage 170 n'est pas bloqué.

On peut par ailleurs prévoir de remplacer les assemblages de serrage 170, 180 et 190 par des dispositifs à limitation de serrage et de desserrage tels que décrits en regard de la figure 4. On pourrait également, dans la forme d'exécution des figures 6 à 8, utiliser un organe de renvoi angulaire qui viendrait s'insérer entre la mâchoire 160 et l'organe de déflection 140.

## Revendications

1. Bride de fixation d'un groupe de fiches et de positionnement angulaire de celles-ci par rapport à une barre de fixation ou d'écartement (1,100), comportant :
- un étau (4; 20,30; 120,130) de serrage des fiches,
- une mâchoire (6,60,160) de positionnement le long de la barre,
- un organe de déflection (40,140) disposé entre l'étau et la mâchoire et comportant sur une face des moyens de rotation de l'étau par rapport à la mâchoire, et sur la face opposée des moyens arqués de guidage du pivotement de l'étau par rapport à la mâchoire selon un axe de pivotement orthogonal à l'axe de rotation et
- des moyens de blocage dans la position désirée,
lesdits moyens de rotation (41,143) coopèrant avec des moyens complémentaires (36,165) de l'étau ou de la mâchoire pour assurer la libre rotation de 0 à 360° de l'étau par rapport à la mâchoire selon un axe de rotation (8) croisant à 90° l'axe de la barre, lesdits moyens arqués de guidage (47,49;141) coopèrant avec des moyens arqués complémentaires (51,137) de la mâchoire ou de l'étau respectivement, et lesdits moyens de blocage comportant un assemblage de serrage (80;98,99;180) assurant simultanément le blocage des fiches d'une part et le blocage dudit pivotement ou de ladite rotation d'autre part dans la position finale désirée.

2. Bride selon la revendication 1, caractérisée en ce que l'étau (4;20,30;120,130) est constitué par un mors d'étau supérieur (20,120) et un mors d'étau inférieur (30,130) présentant un passage central (21,31;121,131) pour ledit assemblage de serrage (80;98,99;180).

3. Bride selon la revendication 1, caractérisée en ce que la mâchoire (6,60,160) est apte à recevoir un dispositif de positionnement et de blocage (7,70,170) le long de la barre (1,100).

4. Bride selon la revendication 1, caractérisée en ce qu'un renvoi angulaire (9) est monté entre la mâchoire (6,60,160) et l'étau (4;20,30;120,130).

5. Bride selon la revendication 4, caractérisée en ce que les surfaces des mors (20,30;120,130) de l'étau situées en vis-à-vis comportent des rainures parallèles (22,32;122,132) pour le logement des fiches (5).

6. Bride selon la revendication 4, caractérisée en ce que l'un des mors (20,30;120,130) de l'étau comporte des saillies (24,136) destinées à pénétrer dans des dégagements correspondants (34,124) de l'autre, pour empêcher la rotation des mors entre eux.

7. Bride selon la revendication 1, caractérisée en ce que l'organe arqué de déflection (40) possède un épaulement tronconique (41) destiné à coopérer, au moins indirectement, avec un évidement tronconique (36) correspondant pratiqué dans l'étau de serrage des fiches, lesdits épaulement et évidement tronconiques présentant un passage central pour un arbre de serrage et en ce que ledit organe arqué de déflection possède des moyens arqués de guidage situés dans des plans parallèles à la barre, destinés à coopérer, au moins indirectement, avec la mâchoire de positionnement le long de la barre.

8. Bride selon la revendication 7, caractérisée en ce que la mâchoire (6) comporte deux parties symétriques séparées par un pont (50) qui présente une ouverture centrale oblongue (53) dans l'axe de la barre (1) et des formes (55, 56) aptes à coopérer avec des formes complémentaires (63, 65) de la mâchoire (6).

9. Bride selon la revendication 8, caractérisée en ce que les moyens de guidage sont constitués par au moins un patin arqué (47) apte à glisser le long d'une creusure (51), de courbure correspondante, pratiquée dans le pont (50) disposé entre les mâchoires.

10. Bride selon la revendication 8, caractérisée en ce que le pont (50) et/ou les deux parties formant la mâchoire (6) présentent des formes (54, 64) correspondant à la section polygonale (101 à 104) de la barre (1).

11. Bride selon la revendication 8, caractérisée en ce que le pont (50) et/ou les deux parties formant la mâchoire (6) présentent des formes (54, 64) correspondant à la section polygonale d'une pièce intermédiaire (110) présentant un passage central de section circulaire apte à recevoir une barre de fixation.

12. Bride selon la revendication 1, caractérisée en ce que l'organe arqué de déflection (140) possède une paroi cylindrique (143) destinée à coopérer avec un rebord correspondant (165) de la mâchoire (160), et en ce que ledit organe arqué de déflection présente une calotte arquée (141) destinée à coopérer avec une creusure (137) de courbure correspondante du mors inférieur (130), ladite creusure présentant une ouverture oblongue (142).

13. Bride selon la revendication 12, caractérisée en ce que la paroi (143) comporte une fente (144) disposée en prolongement de l'une des extrémités de l'ouverture (142), de manière à constituer deux ailes (145,146) destinées à recevoir un assemblage (190) de serrage de l'organe arqué de déflection sur la mâchoire.

14. Bride selon la revendication 13, caractérisée en ce que le rebord (165) comporte sur une partie de sa circonférence une gorge extérieure (167), pour le passage de l'assemblage de serrage (190) solidarisant l'organe de déflection (140) de la mâchoire (160), de manière à limiter la rotation de l'organe de déflection par rapport à la mâchoire sur ladite partie de circonférence.

15. Bride selon la revendication 12, caractérisée en ce que la mâchoire (160) de positionnement de la bride le long de la barre (100) comporte un passage transversal (161) de forme correspondant à celle de la barre et prolongé par une fente (162) délimitant deux ailes (164,164) destinées à recevoir un dispositif de serrage (170)

16. Bride selon la revendication 15 et l'une des revendications 2, 4 ou 13, caractérisée en ce que l'un au moins des dispositifs de serrage (7,70,170; 80,180; 190) comporte un limiteur de vissage et de dévissage.

17. Bride selon la revendication 16, caractérisée en ce que le dispositif de serrage comporte un arbre creux (70) destiné à recevoir par vissage un arbre central (77), lesdits arbres présentant chacun une tête de serrage (71).

18. Bride selon la revendication 17, caractérisée en ce que ledit limiteur de vissage et de dévissage est constitué par une liaison entre lesdits arbres (70 et 77) et par un anneau de type O-ring (74) monté dans une rainure circulaire extérieure (73) de chacune des têtes (71).

19. Bride selon la revendication 18, caractérisée en ce que la liaison entre les arbres est constituée par une vis (79) dimensionnée de telle sorte qu'un intervalle i subsiste entre l'extrémité de l'arbre creux (70) et la face interne de la tête de l'arbre central (77), dans la position dans laquelle la bride coulisse le long de la barre (1).

20. Bride selon la revendication 4, caractérisée en ce que l'assemblage de serrage (80) apte à positionner les fiches comporte un arbre creux (42) solidaire de l'organe arqué de déflection (40) et destiné à coopérer avec des moyens de serrage (81,82).

21. Bride selon la revendication 4, caractérisée en ce que l'assemblage de serrage (80) apte à immobiliser l'organe de déflection (40) comporte un tube central (83) de liaison entre le pont (50) et une vis (88).

22. Bride selon les revendications 3 et 7, caractérisée en ce que ledit renvoi angulaire (9) présente une ouverture tronconique (92) et un épaulement tronconique (97) situés dans des plans différents et aptes à coopérer respectivement avec l'épaulement tronconique (41) et le dégagement tronconique (36).

## Claims

1. Clip for fastening and angularly positioning a set of bone pins with respect to a fixation or extension bar (1,100), said clip comprising:
- a clamp (4; 20,30; 120,130) for gripping the bone pins,
- a positioning jaw (6,60,160) along the bar,
- a deflection member (40,140) located between the clamp and the jaw and having on a face rotation means of the clamp with respect to the jaw and on the opposite face arcuate guiding means for pivoting the clamp with respect to the jaw according to a pivoting axis which is orthogonal with respect to the rotation axis, and
- fastening means in the desired position,
said rotation means (41,143) cooperating with complementary means (36,165) of the clamp or the jaw to ensure a free rotation from 0 to 360° of the clamp with respect to the jaw according to a rotation axis (8) crossing at 90° the axis of the bar, said arcuate guiding means (47,49; 141) cooperating with complementary arcuate means (51,137) of the jaw or of the clamp respectively, and said fastening means having a clamping arrangement (80;98,99;180) simultaneously ensuring the gripping of the pins on the one hand and the fastening of said pivoting or said rotation on the other hand in the final wanted position.

2. Clip according to claim 1, characterised in that the clamp (4; 20,30; 120,130) comprises an upper clamp grip (20,120) and a lower clamp grip (30,130) which have a central passage (21,31;121,131) for said clamping arrangement (80;98,99;180).

3. Clip according to claim 1, characterised in that the positioning jaw (6,60,160) receives a positioning and clamping device (7,70,170) along the bar (1,100).

4. Clip according to claim 1, characterised in that an angle drive means (9) is disposed between the jaw (6,60, 160) and the clamp (4; 20,30; 120,130).

5. Clip according to claim 4, characterised in that those surfaces of the clamp grips (20,30;120,130) which face one another are provided with parallel grooves (22,32;122,132) for the passage of the pins (5).

6. Clip according to claim 4, characterised in that one of the clamp grips (20,30;120,130) is provided with projections (24,136) intended to penetrate into corresponding recesses (34,124) in the other in order to prevent the rotation of the grips relative to each other.

7. Clip according to claim 1, characterised in that the curved deflection member (40) is provided with a frustoconical shoulder (41) intended for at least indirect cooperation with a corresponding frustoconical recess (36) in the pin holder clamp, said frustoconical shoulder and recess having a central passage for a clamp shaft, and in that said curved deflection member is provided with curved guide means situated in planes parallel to the bar and intended for at least indirect cooperation with the jaw effecting positioning along the bar.

8. Clip according to claim 7, characterised in that the jaw (6) effecting positioning along the bar (1) is a clam-shell type jaw having two symmetrical parts separated by a bridge (50) having a central oblong opening (53) in the axis of the bar (1), and shapes (55,56) adapted to cooperate with complementary shapes (63,65) of the jaw (6).

9. Clip according to claim 8, characterised in that the guide means consist of at least one curved skid (47) adapted to slide along a hollow (51) of corresponding curvature formed in the bridge (50) disposed between the jaw parts.

10. Clip according to claim 8, characterised in that the bridge (50) and/or the jaw parts (6) has or have shapes (54,64) corresponding to the polygonal section (101 to 104) of the bar (1).

11. Clip according to claim 8, characterised in that the bridge (50) and/or the jaw parts (6) has or have shapes (54,64) corresponding to the polygonal section of an intermediate part (110) having a central passage adapted to receive a fixation bar.

12. Clip according to claim 1, characterized in that the curved deflection member (140) possesses a cylindrical wall (143) intended for cooperation with a corresponding shoulder (165) in the jaw (160), and in that said deflection member presents a curved cover (141) intended for cooperation with a hollow (137) of corresponding curvature in the lower grip (130), said hollow having an oblong opening (142).

13. Clip according to claim 12, characterised in that the wall (143) presents a slot (144) extending from one of the ends of the opening (142), in order to form two wings (145,146) intended to receive a fixation arrangement (190) for clamping the curved deflection member on the jaw.

14. Clip according to claim 13, characterised in that the shoulder (165) presents an external groove (167) on a portion of its circumberence, intended to receive the fixation arrangement (190) which bounds the deflection member (140) with the jaw (160), in order to limit the rotation of the deflection member with respect to the jaw on said circumferential portion.

15. Clip according to claim 12, characterised in that the jaw (160) for positioning the clip along the bar (100) has a transverse passage (161) having a shape corresponding to that of the bar, and a slot (162) extending from said passage, in order to form two wings (164,165) intended to receive a clamping arrangement (170).

16. Clip according to claim 15 and one of the claims 2, 4 or 13, characterised in that at least one of the arrangements (7,70,170; 80,180; 190) is provided with a screwing and unscrewing limiter.

17. Clip according to claim 16, characterised in that said arrangement comprises a hollow shaft (70) intended to receive screwthreadedly a central shaft (77), each of said shafts having a clamping head (71).

18. Clip according to claim 17, characterised in that said screwing and unscrewing limiter consists of a connection between said shafts (70 and 77) and of a ring (74) of the O-ring type mounted in an external circular groove (73) in each of the heads (71).

19. Clip according to claim 18, characterised in that the connection between the shafts consists of a screw (79) of such dimensions that a gap (i) is left between the end of the hollow shaft (70) and the inner face of the head of the central shaft (77) in the position in which the clip slides along the bar (1).

20. Clip according to claim 4, characterised in that the clamping arrangement (80) adapted to position the pins comprises a hollow shaft (42) fastened to the curved deflection member (40) and intended to cooperate with clamping means (81,82).

21. Clip according to claim 4, characterised in that the clamping arrangement (80) adapted to lock the deflection member (40) in position comprises a central tube (83) making the connection between the bridge (50) and a screw (88).

22. Clip according to claims 3 and 7, characterised in that said angle drive means (9) has a frustoconical opening (92) and a frustoconical shoulder (97) which are situaded in different planes and are adapted to cooperate respectively with the frustoconical shoulder (41) and the frustoconical recess (36).

## Patentansprüche

1. Befestigungskörper für eine Gruppe Stifte und zum winkelmäßigen Positionieren derselben in bezug auf eine Befestigungs- oder Abstandhalterstange (1, 100), mit:
- einem Schraubstock (4; 20, 30; 120, 130), zum Einspannen der Stifte,
- einer Klemme (6, 60, 160) für die Positionierung entlang der Stange,
- einem Ablenkteil (40, 140), das zwischen dem Schraubstock und der Klemme angeordnet ist und auf einer Fläche Einrichtungen zum Drehen des Schraubstocks in bezug auf die Klemme und an der gegenüberliegenden Fläche bogenförmige Führungseinrichtungen für das Verschwenken des Schraubstocks in bezug auf die Klemme entlang einer Schwenkachse, die orthogonal zu der Drehachse liegt, umfaßt, und
- Einrichtungen für das Blockieren in der gewünschten Position,
wobei die Dreheinrichtungen (41, 143), die mit den komplementären Einrichtungen (36, 165) des Schraubstockes oder der Klemme zusammenwirken, um die freie Drehung zwischen 0 und 360° des Schraubstockes in bezug auf die Klemme entlang einer Drehachse (8), die die Achse der Stange unter 90° schneidet, zu gewährleisten, die bogenförmigen Führungseinrichtungen (47, 49; 141), die mit den komplementären bogenförmigen Einrichtungen (51, 137) der Klemme oder des Schraubstockes jeweils zusammenwirken, und die Blockiereinrichtungen, die eine Einspannanordnung (80; 98, 99; 180) aufweisen, gleichzeitig die Blockierung der Stifte einerseits und die Blockierung der Schwenkbewegung oder der Drehung andererseits in der gewünschten endgültigen Position sicherstellen.

2. Körper gemäß Anspruch 1, dadurch gekennzeichnet, daß der Schraubstock (4; 20, 30; 120, 130) durch eine obere Schraubstock-Spannbacke (20, 120) und eine untere Schraubstock-Spannbacke (30, 130) gebildet ist, die einen Mittendurchlaß (21, 31; 121, 131) für die Einspannanordnung (80; 98, 99; 180) aufweisen.

3. Körper nach Anspruch 1, dadurch gekennzeichnet, daß die Klemme (6, 60, 160) dazu ausgelegt ist, eine Vorrichtung zum Positionieren und zum Blockieren (7, 70, 170) entlang der Stange (1, 100) aufzunehmen.

4. Körper nach Anspruch 1, dadurch gekennzeichnet, daß eine Winkelrückstelleinrichtung (9) zwischen der Klemme (6, 60, 160) und dem Schraubstock (4; 20, 30; 120, 130) angebracht ist.

5. Körper nach Anspruch 4, dadurch gekennzeichnet, daß die Flächen der Spannbacken (20, 30; 120, 130) des Schraubstockes, die einander gegenüberliegen, parallele Rillen (22, 32; 122, 132) für das Unterbringen der Stifte (5) aufweisen.

6. Körper nach Anspruch 4, dadurch gekennzeichnet, daß eine der Spannbacken (20, 30; 120, 130) des Schraubstockes Vorsprünge (24, 136) aufweist, die dazu bestimmt sind, in entsprechende Ausnehmungen (34, 124) der anderen einzudringen, um die Drehung der Spannbacken untereinander zu verhindern.

7. Körper nach Anspruch 1, dadurch gekennzeichnet, daß das bogenförmige Ablenkteil (40) eine kegelstumpfförmige Schulter (41) besitzt, dazu bestimmt, zumindest indirekt mit einer kegelstumpfartigen Ausnehmung (36) zusammenzuwirken, die entsprechend in dem Schraubstock für das Einspannen der Stifte angebracht ist, wobei die kegelstumpfartige Schulter und Ausnehmung einen Mittendurchlaß für eine Einspannwelle bilden, und daß das bogenförmige Ablenkteil bogenförmige Führungseinrichtungen besitzt, die in den zur Stange parallelen Ebenen angeordnet sind, dazu bestimmt, zumindest indirekt mit der Klemme zum Positionieren entlang der Stange zusammenzuwirken.

8. Körper nach Anspruch 7, dadurch gekennzeichnet, daß die Klemme (6) zwei symmetrische Teile, die durch eine Brücke (50) getrennt sind, welche eine längliche Mittenöffnung (53) in der Achse der Stange (1) ausbildet, und Formen (55, 56), die dazu ausgelegt sind, mit komplementären Formen (63, 65) der Klemme (6) zusammenzuwirken, aufweist.

9. Körper nach Anspruch 8, dadurch gekennzeichnet, daß die Führungseinrichtungen durch wenigstens eine bogenförmige Kufe (47) gebildet sind, die dazu ausgelegt ist, entlang einer Einsenkung (51) entsprechender Krümmung zu gleiten, die in der Brücke (50) ausgebildet ist, die zwischen den Klemmen angeordnet ist.

10. Körper nach Anspruch 8, dadurch gekennzeichnet, daß die Brücke (50) und/oder die beiden Teile, die die Klemme (6) bilden, Formen (54, 64) zeigen, die dem polygonalen Querschnitt (101 bis 104) der Stange (1) entsprechen.

11. Körper nach Anspruch 8, dadurch gekennzeichnet, daß die Brücke (50) und/oder die beiden Teile, die die Klemme (6) bilden, Formen (54, 64) zeigen, die dem polygonalen Querschnitt eines Zwischenstückes (110) entsprechen, welches einen Mittendurchlaß mit kreisförmigem Querschnitt bildet, dazu ausgelegt, eine Befestigungsstange aufzunehmen.

12. Körper nach Anspruch 1, dadurch gekennzeichnet, daß das bogenförmige Ablenkteil (140) eine zylindrische Wand (143) besitzt, dazu bestimmt, mit einer entsprechenden Leiste (165) der Klemme (160) zusammenzuwirken, und daß das bogenförmige Ablenkteil eine bogenförmige Krone (141) zeigt, dazu bestimmt, mit einer Einsenkung (137) entsprechender Krümmung der unteren Spannbacke (130) zusammenzuwirken, wobei die Einsenkung eine längliche Öffnung (142) zeigt.

13. Körper nach Anspruch 12, dadurch gekennzeichnet, daß die Wand (143) einen Spalt (144) aufweist, der in Verlängerung des einen der Enden der Öffnung (142) angeordnet ist, derart, daß zwei Flügel (145, 146) gebildet sind, dazu bestimmt, eine Einspannanordnung (190) des bogenförmigen Ablenkteils auf der Klemme aufzunehmen.

14. Körper nach Anspruch 13, dadurch gekennzeichnet, daß die Leiste (165) auf einem Teil seines Umfangs eine äußere Kehle (167) aufweist, für den Durchlaß der Einspannanordnung (190), einstückig ausgebildet mit dem Ablenkteil (140) der Klemme (160), derart, daß die Drehung des Ablenkteils in bezug auf die Klemme auf dem Umfangsteil begrenzt wird.

15. Körper nach Anspruch 12, dadurch gekennzeichnet, daß die Klemme (116) zum Positionieren des Körpers entlang der Stange (100) einen Querdurchlaß (116) einer Form entsprechend der der Stange und durch einen Spalt (162) verlängert, der zwei Flügel (164, 164) begrenzt, die dazu bestimmt sind, eine Einspannvorrichtung (170) aufzunehmen, aufweist.

16. Körper nach Anspruch 15 und einem der Ansprüche 2, 4 oder 13, dadurch gekennzeichnet, daß wenigstens eine der Einspannvorrichtungen (7, 70, 170; 80, 180; 190) einen Schraub- und Aufschraubbegrenzer aufweist.

17. Körper nach Anspruch 16, dadurch gekennzeichnet, daß die Einspannvorrichtung eine Hohlwelle (70) aufweist, dazu bestimmt, durch Einschrauben eine Mittenwelle (77) aufzunehmen, wobei die Wellen jede einen Einspannkopf (71) aufweisen.

18. Körper nach Anspruch 17, dadurch gekennzeichnet, daß der Schraub- und Aufschraubbegrenzer durch eine Verbindung zwischen den Wellen (70 und 77) und durch einen Ring vom Typ des O-Ringes (74), der in einer äußeren kreisförmigen Rille (73) jeder der Köpfe (71) angeordnet ist, gebildet ist.

19. Körper nach Anspruch 18, dadurch gekennzeichnet, daß die Verbindung zwischen den Wellen durch eine Schraube (79) gebildet ist, die derart bemessen ist, daß zwischen dem Ende der Hohlwelle (70) und der Innenfläche des Kopfes der Mittenwelle (77) ein Intervall i vorhanden ist, in der Position, in der der Körper entlang der Stange (1) gleitet.

20. Körper nach Anspruch 4, dadurch gekennzeichnet, daß die Einspannanordnung (80), die dazu ausgelegt ist, die Stifte zu positionieren, eine Hohlwelle (42) aufweist, die einstückig mit dem bogenförmigen Ablenkteil (40) ausgebildet ist und dazu bestimmt ist, mit den Einspanneinrichtungen (81, 82) zusammenzuwirken.

21. Körper nach Anspruch 4, dadurch gekennzeichnet, daß die Einspannanordnung (80), die dazu ausgelegt ist, das Ablenkteil (40) unbeweglich zu halten, ein Mittelrohr (43) für die Verbindung zwischen der Brücke (50) und einer Schraube (88) aufweist.

22. Körper nach den Ansprüchen 3 und 7, dadurch gekennzeichnet, daß die Winkelrückstelleinrichtung eine kegelstumpfförmige Öffnung (92) und eine kegelstumpfförmige Schulter (97) zeigt, die in unterschiedlichen Ebenen liegen und dazu ausgelegt sind, jeweils mit der kegelstumpfförmigen Schulter (51) und der kegelstumpfförmigen Ausnehmung (36) zusammenzuwirken.
